(19) Europäisches Patentamt
European Patent Office
Office européen des brevets

(11) Numéro de publication: **0 526 302 B1**

(12) **FASCICULE DE BREVET EUROPEEN**

(45) Date de publication de fascicule du brevet: **01.03.95**

(51) Int. Cl.[6]: **A61K 7/48**, C07C 69/732, C07C 235/34

(21) Numéro de dépôt: **92402078.7**

(22) Date de dépôt: **17.07.92**

(54) **Composition à action dépigmentante à base de dérivés d'acide (2,5-dihydroxyphenyl) carboxylique.**

(30) Priorité: **17.07.91 FR 9109029**

(43) Date de publication de la demande:
**03.02.93 Bulletin 93/05**

(45) Mention de la délivrance du brevet:
**01.03.95 Bulletin 95/09**

(84) Etats contractants désignés:
**AT BE CH DE DK ES FR GB GR IT LI NL PT SE**

(56) Documents cités:
**EP-A- 0 069 068**
**WO-A-82/04189**
**FR-A- 2 400 359**
**FR-A- 2 401 900**

(73) Titulaire: **L'OREAL**
**14, Rue Royale**
**F-75008 Paris (FR)**

(72) Inventeur: **Junino, Alex**
**162, avenue Vauban**
**F-93190 Livry Gargan (FR)**
Inventeur: **Lagrange, Alain**
**5, rue Montry**
**F-77700-Coupvray (FR)**
Inventeur: **N'Guyen, Ouang Lan**
**45, avenue Alsace Lorraine**
**F-92160 Antony (FR)**
Inventeur: **Bourboulon, Marie-Alix**
**68, rue d'Alleray**
**F-75015 Paris (FR)**

(74) Mandataire: **Stalla-Bourdillon, Bernard et al**
**Cabinet Nony**
**29, rue Cambacérès**
**F-75008 Paris (FR)**

EP 0 526 302 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet l'utilisation de dérivés d'acides (2,5-dihydroxyphényl)carboxylique, leurs homologues et leurs sels dans des compositions cosmétiques ou dermatologiques par application topique dans le but de blanchir la peau ou de traiter les taches pigmentaires ainsi que de nouveaux homologues d'acides (2,5-dihydroxyphényl)carboxyliques.

On rappellera que le mécanisme de formation de la pigmentation de la peau, c'est-à-dire de la formation des mélanines est particulièrement complexe et fait intervenir schématiquement les principales étapes suivantes :

Tyrosine ---> Dopa ---> Dopaquinone ---> Dopachrome ---> Mélanines

l'enzyme intervenant dans cette suite de réactions étant essentiellement la tyrosinase.

Les substances les plus utilisées à l'heure actuelle en tant que dépigmentants sont plus particulièrement l'hydroquinone et ses dérivés en particulier ses éthers tels que le monométhyléther d'hydroquinone.

Ces composés, s'ils ont une efficacité certaine, ne sont malheureusement pas exempts d'effets secondaires, ce qui peut rendre leur emploi délicat voir dangereux.

Ainsi l'hydroquinone, dont l'emploi est d'ailleurs limité à une concentration de 2 %, est un composé particulièrement irritant et cytotoxique pour le mélanocyte, dont le remplacement, total ou partiel a été envisagé par de nombreux auteurs.

Il a ainsi été proposé dans le brevet US n° 4 526 179 certains esters gras d'hydroquinone ayant une bonne activité et étant moins irritants et plus stables que l'hydroquinone.

De même on a proposé d'autres dérivés d'hydroquinone dans la demande japonaise n° 27909/86 ne présentant pas les inconvénients de l'hydroquinone mais dont l'efficacité s'est révélée relativement médiocre.

Il est bien établi qu'une substance exerce une action dépigmentante si elle agit directement sur la vitalité des mélanocytes épidermiques où se déroule normalement la mélanogénèse, et/ou si elle interfère avec une des étapes de la biosynthèse des mélanines soit en inhibant un des enzymes impliqués soit en s'intercalant comme analogue structural dans la voie de synthèse qui peut ainsi être bloquée d'où l'effet dépigmentant.

L'utilisation de substances dépigmentantes topiques présentant une bonne efficacité et étant inoffensive, est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou secondaire à la contraception oestro-progestative, les hyperpigwentations localisées par hyperactivité et prolifération mélanocytaire bénigne telles que les taches pigmentaires séniles dites lentigo actiniques, les hyperpigmentations accidentelles telles que la photosensibilisation et la cicatrisation post-lésionnelle, ainsi que certaines leucodermies telles que le vitiligo ou, à défaut de pouvoir repigmenter la peau lésée, on achève de dépigmenter les zones de peau normale résiduelle pour donner à l'ensemble de la peau une teinte blanche homogène.

Après de nombreuses études sur différentes substances on a constaté de façon tout à fait surprenante que certains dérivés d'acides (2,5-dihydroxyphényl)carboxyliques, leurs homologues et leurs sels avaient une action dépigmentante particulièrement prononcée nettement supérieure à celle de l'hydroquinone dans le test d'inhibition "in vitro" de l'activité de la tyrosinase comme ceci sera décrit ci-après.

La présente invention a donc pour objet l'utilisation de dérivés d'acides (2,5-dihydroxyphényl)-carboxyliques, leurs homologues et leurs sels pour la préparation d'une composition cosmétique ou dermatologique ayant une action dépigmentante, lesdits dérivés répondant à la formule générale suivante :

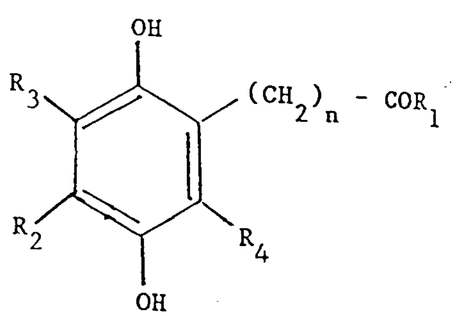

(I)

dans laquelle :
$R_1$ représente $OR_5$, OH ou

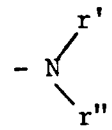

$R_5$ représente un radical alkyle en $C_1$ - $C_{20}$, linéaire ou ramifié, un radical alcényle en $C_2$-$C_{20}$ linéaire ou ramifié, un radical alkyle en $C_1$ - $C_{20}$ substitué par un ou plusieurs groupements hydroxy ou alcoxy,

r' et r'', identiques ou différents, représentent un atome d'hydrogène, un radical alkyle en $C_1$ - $C_{20}$, un radical hydroxyalkyle en $C_2$ - $C_6$ ou un radical polyhydroxyalkyle en $C_3$ - $C_6$ ou r' et r'' pris ensemble, avec l'atome d'azote, forment un hétérocycle,
le nombre d'atomes de carbone de la chaîne $-(CH_2)_n-COR_1$ étant inférieur ou égal à 21,
$R_2$ et $R_3$, identiques ou différents représentent un atome d'hydrogène, un radical alkyle en $C_1$ - $C_4$, linéaire ou ramifié, ou un alcoxy en $C_1$ - $C_4$,
$R_4$ représente un atome d'hydrogène ou un radical alkyle en $C_1$ - $C_4$, linéaire ou ramifié, et
n est 0 à 20, sous réserve que lorsque $R_2$ et $R_3$ représentent un atome d'hydrogène n est supérieur ou égal à 2.

Par radical alkyle en $C_1$ - $C_{20}$, on doit entendre de préférence un radical méthyle, éthyle, propyle, isopropyle, butyle, tertiobutyle, isoamyle, octyle, éthyl-2 hexyle, dodécyle, tétradécyle, hexadécyle ou octadécyle.

Par radical alcényle en $C_2$-$C_{20}$, on doit entendre de préférence le radical oléyle.

Par radical alkyle en $C_1$-$C_{20}$ substitué par un ou plusieurs groupements hydroxy ou alcoxy, on doit entendre notamment un radical hydroxyméthyle, 2-hydroxyéthyle, 2-hydroxypropyle, 3-hydroxypropyle, 4-hydroxybutyle, 5-hydroxypentyle, 6-hydroxyhexyle, méthoxyéthyle, ou éthoxyméthyle.

Par radical hydroxyalkyle en $C_2$-$C_6$, on doit notamment entendre un radical 2-hydroxyéthyle, 2-hydroxypropyle ou 3-hydroxypropyle.

Par radical polyhydroxyalkyle en $C_3$ - $C_6$, on doit notamment entendre un radical ayant de 3 à 6 atomes de carbone et de 2 à 5 groupes hydroxyles tel que le radical 2,3-dihydroxypropyle, 2,3,4-trihydroxybutyle ou 2,3,4,5-tétrahydroxypentyle.

Par radical alcoxy en $C_1$ - $C_4$, on doit notamment entendre un radical méthoxy, éthoxy, propoxy, isopropoxy ou butoxy.

Lorsque r' et r'' pris ensemble forment avec l'atome d'azote un hétérocycle, celui-ci peut être un cycle morpholino, pyrrolidino, pipéridino ou pipérazino éventuellement substitué par un radical alkyle ou hydroxyalkyle en $C_1$ - $C_4$.

Parmi les composés de formule générale (I), on peut citer les composés suivants :

- l'acide 2,5-dihydroxyphényl propionique et ses esters éthylique et laurique,
- l'acide 2,5-dihydroxy 3,4-diméthylphényl acétique et son ester éthylique,
- le 2,5-dihydroxy 4-méthyl phényl acétate de méthyle,
- l'acide 2,5-dihydroxy 4-méthyl phényl acétique,
- l'acide 2,5-dihydroxy 4-méthyl phényl propionique et son ester éthylique,
- l'acide 2,5-dihydroxy-4-méthyl benzoïque et son ester méthylique ou éthylique,
- l'acide 2,5-dihydroxy-4-éthyl benzoïque,
- l'acide 2,5-dihydroxy-4-méthoxy benzoïque et son ester méthylique,
- l'acide 2,5-dihydroxy-4-éthoxy benzoïque,
- le 3-(2,5-dihydroxy 4'-méthyl phényl)-1-N(ω-carboxydécyl) propylamide,
- l'acide 2,5-dihydroxy 4-méthyl phényl butanoïque,
- l'acide 2,5-dihydroxy 4-méthyl phényl hexanoïque,
- l'acide 2,5-dihydroxy 4-méthoxy phényl acétique et son ester méthylique,
- le 2,5-dihydroxy 4-méthoxy benzylamide,
- l'ester méthylique de l'acide 2,5-dihydroxy 3-méthoxy phényl acétique,
- l'acide 2,5-dihydroxy 3-méthoxy phényl pentadécylique, et son ester méthylique,
- l'acide 2,5-dihydroxy phényl butanoïque et son ester méthylique,
- le 2,5-dihydroxy phényl butylamide,
- l'acide 2,5-dihydroxy phényl pentanoïque,
- le 2,5-dihydroxy phényl pentylamide,

- l'acide 2,5-dihydroxy phényl hexanoïque,
- l'acide 2,5-dihydroxy phényl octanoïque,
- l'acide 2,5-dihydroxy phényl décylique et son ester éthylique,
- l'acide 2,5-dihydroxy phényl undécylique et son ester méthylique,
- l'acide 2,5-dihydroxy-3,4-diméthyl phényl butanoïque,
- l'acide 2,5-dihydroxy-3,4-diméthoxy phényl acétique,
- l'ester éthylique de l'acide 2,5-dihydroxy 4,6-diméthyl phényl acétique,
- le 2-(2,5-dihydroxy 4-méthyl phényl)-N-octyl acétamide, et
- l'acide 6-(2,5-dihydroxy 4-méthoxy phényl) hexanoïque.

Selon une forme de réalisation préférée de l'invention, les dérivés d'acides (2,5-dihydroxyphényl)-carboxyliques, leurs homologues et leurs sels répondent à la formule générale (II) suivante :

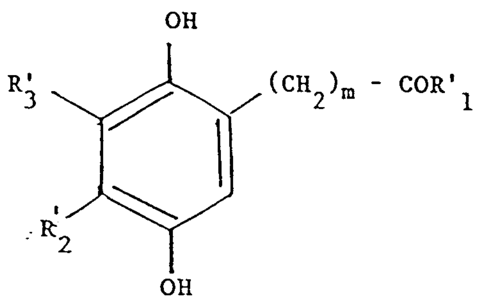

(II)

dans laquelle :

R'$_1$ a la même signification que donnée ci-dessus pour $R_1$ selon la formule (I),

m est 1 ou 2,

(i) lorsque m est 1, l'un au moins des radicaux R'$_2$ et R'$_3$ représente un radical alkyle en $C_1$ - $C_4$ linéaire ou ramifié, l'autre représentant éventuellement un atome d'hydrogène,

(ii) lorsque m est 2, R'$_2$ et R'$_3$, identiques ou différents, représentent un atome d'hydrogène ou un radical alkyle en $C_1$ - $C_4$ linéaire ou ramifié.

Parmi les composés particulièrement préférés on peut notamment citer :

l'acide 2,5-dihydroxyphényl propionique,

l'acide 2,5-dihydroxy 3,4-diméthylphényl acétique,

le 2,5-dihydroxy 4-méthyl phényl acétate de méthyle,

l'acide 2,5-dihydroxy 4-méthyl phényl acétique, et

l'acide 2,5-dihydroxy 4-méthyl phényl propionique.

Dans les compositions dépigmentantes selon l'invention la concentration en dérivés d'acides (2,5-dihydroxyphényl)carboxyliques, leurs homologues et leurs sels de formule (I) est généralement comprise entre 0,01 et 10 % et de préférence entre 0,5 et 5 % en poids par rapport au poids total de la composition.

Le véhicule des compositions peut être notamment une solution aqueuse ou hydroalcoolique, une émulsion du type huile-dans-l'eau ou eau-dans-l'huile, un gel émulsionné ou encore un système biphasé.

De préférence les compositions selon l'invention se présentent sous forme d'une lotion, d'une crème, d'un lait, d'un gel, d'un masque, de microsphères ou nanosphères ou de dispersions vésiculaires. Dans le cas des dispersions vésiculaires, les lipides constitutifs des vésicules peuvent être du type ionique ou non ionique ou bien un mélange de ceux-ci.

Ces compositions cosmétiques peuvent également contenir un humectant, un agent de surface, un kératolytique, un agent anti-inflammatoire, un agent complexant, un anti-oxydant, un conservateur, un parfum ou un filtre solaire.

Ces compositions sont appliquées par voie topique en quantité correspondant aux doses d'application usuelles pour le type de composition considéré (gel, crème, lotion, et...). Par exemple dans le cas d'une crème on utilise de 0,5 à 3 mg et notamment de 1 à 2 mg de crème par $cm^2$ de peau et par application, à raison d'une ou deux applications par jour.

Les dérivés d'acides (2,5-dihydroxyphényl)carboxyliques de formule générale (I) sont pour la plupart connus et ont été décrits notamment dans les brevets français n° 7824174 (2 400 358) et n° 7824175 (2 400 359).

La présente invention a également pour objet à titre de composés nouveaux les dérivés d'acides (2,5-dihydroxyphényl)carboxyliques répondant à la formule générale (III) suivante :

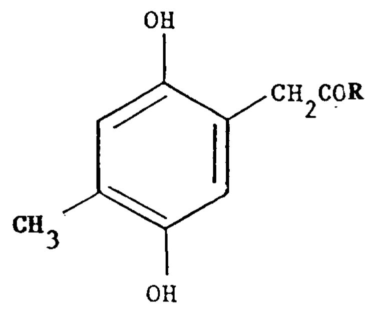

(III)

dans laquelle :
R représente le radical OR' ou NHR'', R' représentant un radical alkyle inférieur en $C_1$-$C_6$, linéaire ou ramifié et R'' représentant un radical alkyle en $C_4$-$C_{12}$.

Parmi les composés répondant à la formule (III), on peut citer les composés suivants :

- le 2,5-dihydroxy 4-méthyl phényl acétate de méthyle,
- le 2,5-dihydroxy 4-méthyl phényl acétate d'éthyle,
- le 2,5-dihydroxy 4-méthyl phényl acétate de propyle,
- le 2,5-dihydroxy 4-méthyl phényl acétate d'isopropyle,
- le 2,5-dihydroxy 4-méthyl phényl acétate de butyle,
- le 2,5-dihydroxy 4-méthyl phényl acétate d'isoamyle, et
- le 2-(2,5-dihydroxy 4-méthyl phényl)-N-octyl acétamide.

Etudes "in vitro"

Certains des dérivés d'acides (2,5-dihydroxyphényl)carboxyliques, leurs homologues et leurs sels de formule générale (I) ont été étudiés comparativement à l'hydroquinone à quantité molaire équivalente dans le test d'inhibition in vitro de l'activité de la tyrosinase.

Selon ce test on suit par spectrométrie visible à 475 nm la quantité de dopachrome formée au cours de la chaîne de réactions de transformation de la tyrosine en mélanines. Ces réactions sont catalysées in vitro par la tyrosinase de champignons, en présence d'un co-substrat réducteur (par exemple la L-dopa en petite quantité) pour initier la réaction d'hydroxylation de la L-tyrosine en L-dopa, laquelle est ensuite oxydée catalytiquement en dopaquinone puis en dopachrome, produit intermédiaire avant les réactions d'oxydation non enzymatiques aboutissant à la formation des mélanines.

On mesure donc la concentration en dopachrome formé au cours du temps en présence et en absence de l'inhibiteur.

Les concentrations en inhibiteurs sont fixées à 50 % molaire par rapport à la concentration en tyrosine dans le milieu réactionnel.

On exprime l'effet d'inhibition par l'abaissement de la quantité maximale de dopachrome formée (valeur de densité optique à 475 nm lue au plateau de la courbe) par rapport à la quantité obtenue en l'absence d'inhibiteur.

Protocole expérimental

Réactifs :

A - Tampon phosphate 0,1 M pH = 6,5 (Tween 20 à 1 %)
B - Solution mère de L-tyrosine à $2.10^{-3}$M dans A
C - Solution mère de L-dopa à $10^{-4}$ M dans A
D - Solution mère de tyrosinase de champignons à 2.400 unités/ml dans A
E - Solution mère de l'inhibiteur à $10^{-2}$M dans A
(les solutions C et D sont à préparer le jour même)

Résultats

- cuve de référence : 3 ml de A
- cuve d'essai :
  - 1 ml de B
  - 0,1 ml de C
  - 1,85ml de A + E
- homogénéiser et équilibrer à 25 °C
- ajouter 0,05 ml de D
- mélanger rapidement et observer la cinétique par la mesure de l'absorbance à 475 nm en fonction du temps.

TABLEAU I

| Composés | % inhibition |
| --- | --- |
| 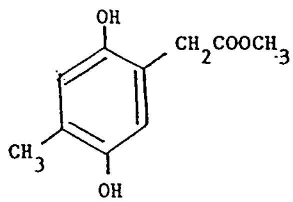 | - 87 % |
| 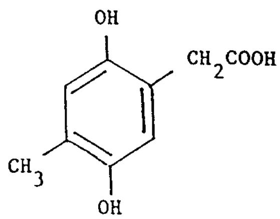 | - 66 % |
| 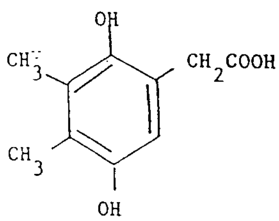 | - 59 % |
| 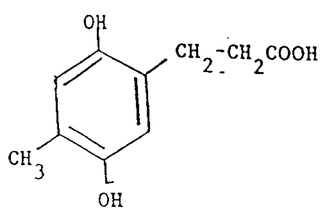 | - 64 % |
| 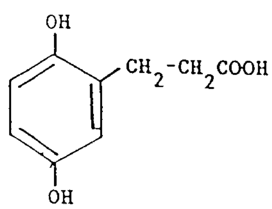 | - 42 % |
| 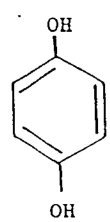<br>(hydroquinone) | - 33 % |

Comme on peut le constater les dérivés d'acides (2,5-dihydroxyphényl)carboxyliques, leurs homologues et leurs sels des compositions selon l'invention ont une activité inhibitrice de la mélanogénèse nettement supérieure à celle de l'hydroquinone.

EXEMPLES DE PREPARATION

EXEMPLE 1 - Préparation du 2,5-dihydroxy 4-méthyl phényl acétate de méthyle

On chauffe à reflux pendant sept heures un mélange de 145,6 g d'acide 2,5-dihydroxy 4-méthyl phényl acétique et 45 g de résine sulfonique acide sèche (IRN-77$^R$) dans 4,38 litres de méthanol absolu. On filtre et le filtrat est évaporé. Le solide blanc est recristallisé d'un mélange d'acétate d'éthyle et d'heptane (50/50) pour conduire à des cristaux blancs ayant les caractéristiques suivantes :

Point de fusion = 138,5 ° C

| Analyse élémentaire : | | | |
|---|---|---|---|
| Calculé<br>Trouvé | C% 61,22<br>61,23 | H% 6,16<br>6,14 | O% 32,62<br>32,40 |

EXEMPLES 2 à 6

Selon le même mode opératoire que celui décrit ci-dessus pour l'exemple 1, mais en remplaçant l'éthanol par l'alcool correspondant, on a préparé les composés suivants :

EXEMPLE 2 - 2,5-dihydroxyphényl-4-méthyl-acetate d'éthyle

Cristaux blancs F = 134 ° C (acétate d'isopropyle)

| Analyse élémentaire : $C_{11}$ $H_{14}$ $O_4$ | | | |
|---|---|---|---|
| Calculé<br>Trouvé | C% 62,85<br>62,79 | H% 6,71<br>6,72 | O% 30,44<br>30,32 |

EXEMPLE 3 - 2,5-dihydroxyphényl-4-méthyl-acétate de propyle

Cristaux blancs F = 101 ° C (éther isopropylique)

| Analyse élémentaire : $C_{12}$ $H_{16}$ $0_4$ | | | |
|---|---|---|---|
| Calculé<br>Trouvé | C% 64,27<br>64,32 | H% 7,19<br>7,22 | O% 28,54<br>28,45 |

EXEMPLE 4 - 2,5-dihydroxyphényl-4-méthyl-acétate d'isopropyle

Cristaux blancs F = 123 ° C (eau)

| Analyse élémentaire : $C_{12}$ $H_{16}$ $0_4$ | | | |
|---|---|---|---|
| Calculé<br>Trouvé | C% 64,27<br>64,13 | H% 7,19<br>7,28 | O% 28,54<br>28,34 |

EXEMPLE 5 - 2,5-dihydroxphenyl-4-méthyl-acétate de butyle

Cristaux blancs F = 113 ° C (eau)

| Analyse élementaire : $C_{13}$ $H_{18}$ $O_4$ | | | |
|---|---|---|---|
| Calculé<br>Trouvé | C% 65,53<br>65,58 | H% 7,61<br>7,60 | O% 26,86<br>27,02 |

EXEMPLE 6- 2,5-dihydroxyphényl-4-méthyl-acétate d'isoamyle

Cristaux blancs F = 99 ° C (acétate d'isopropyle)

| Analyse élémentaire pour $C_{14}$ $H_{20}$ $O_4$ | | | |
|---|---|---|---|
| Calculé<br>Trouvé | C% 66,65<br>66,47 | H% 7,99<br>7,91 | O% 25,36<br>25,09 |

EXEMPLE 7 - Préparation du 2-(2,5-dihydroxy-4-méthyl-phényl)-N-octylacétamide

On introduit une solution de 7,75 gr de n-octyl-amine dissous dans 15 ml de diméthylformamide, dans une solution de 10 gr de 5-hydroxy-6-méthyl-3h-benzofuran-2-one dissous dans 15 ml de diméthylformamide. Après 15 minutes à 100 ° C, on évapore à sec sous pression réduite. On reprend avec 100 ml d'acétate d'éthyle, on lave à l'eau, sèche puis on évapore pour conduire à un solide blanc qui est recristallisé de l'acétate d'isopropyle.

Point de fusion : 118 ° C Cristaux blancs

| Analyse élémentaire : $C_{17}$ $H_{27}$ N $O_3$ | | | | |
|---|---|---|---|---|
| Calculé<br>Trouvé | C% 69,59<br>69,50 | H% 9,28<br>9,15 | N% 4,77<br>4,58 | O% 16,36<br>16,66 |

EXEMPLE 8 - Préparation de l'acide 6-(2,5-dihydroxy-4-méthoxy-phényl)hexanoïque

a) Préparation de l'acide 6-(2,5-dihydroxy-4-méthoxy-phényl)-6-oxo-hexanoïque

On mélange dans un tricol, sous azote, 7 gr de 2-méthoxy-hydroquinone, 13,6 gr du monoesterméthylique de l'acide adipique dans 60 ml de dichloroéthane. On coule 13 ml d'éthérate de trifluorure de bore à température ambiante. Après cinq heures de reflux, on jette le milieu réactionnel dans un mélange de 40 gr d'acétate de sodium trihydraté et de 100 ml d'eau. On extrait avec 500 ml d'acétate d'éthyle, on lave, on sèche, on concentre sous vide. Le résidu est traité par un mélange de 1,4 ml d'acide sulfurique concentré, 14 ml d'eau et de 40 ml d'acide acétique à 80 ° C pendant 3 heures puis la solution est coulée dans 100 ml d'eau glacée. Le précipité est filtré, lavé plusieurs fois avec 20 ml d'eau et séché. Le solide est recristallisé dans un mélange acétate d'éthyle et d'éther isopropylique pour conduire à des cristaux jaunes ayant les caractéristiques suivantes :

Point de fusion : 165 ° C

| Analyse élementaire : $C_{13}$ $H_{16}$ $O_6$ | | | |
|---|---|---|---|
| Calculé<br>Trouvé | C% 58,20<br>58,02 | H% 6,01<br>6,09 | O% 35,78<br>35,73 |

b) Préparation de l'acide 6-(2,5-dihydroxy-4-méthoxy-phényl)hexanoïque

On mélange 2 gr de laine de zinc, 0,13 gr de chlorure mercurique, 0,13 ml d'acide chlorhydrique concentré, 4 ml d'eau sous agitation. Après 10 minutes, on décante la phase aqueuse puis on lave l'amalgame avec de l'eau. A cet amalgame on ajoute une solution de 2,5 gr de l'acide 6-(2,5-dihydroxy-4-méthoxy-phényl)- 6-oxo-hexanoïque dans 20 ml de toluène puis une solution diluée d'acide chlorhydrique (6 ml HCl concentré et 3 ml d'eau). Après 8 heures de reflux, on jette dans 300 ml d'eau glacée. On extrait avec de l'acétate d'éthyle (3 x 100 ml), on lave avec de l'eau, sèche puis on évapore sous vide. Le solide est recristallisé dans un mélange d'acétate d'éthyle et d'heptane pour donner des cristaux blancs ayant les caractéristiques suivantes :

Point de fusion : 142 °C

| Analyse élémentaire : $C_{13}$ $H_{18}$ $0_5$ | | | |
|---|---|---|---|
| Calculé<br>Trouvé | C% 61,41<br>61,33 | H% 7,14<br>7,20 | O% 31,46<br>31,50 |

EXEMPLES DE COMPOSITIONS

EXEMPLE 1 : Emulsion eau-dans-huile

| | |
|---|---|
| - Glycérine | 5 g |
| - Propylène glycol | 10 g |
| - 2,5-dihydroxy 4-méthyl phényl acétate de méthyle | 0,5 g |
| - Cyclométhicone (cyclopentadiméthylsiloxane) | 20 g |
| - Abil W09 (Mélange de polycétyldiméthylsiloxane oxyéthyléné oxypropyléné/isostérate de polyglycéryle à 4 moles de glycérol/laurate d'hexyle) | 3 g |
| - Parfum | 0,1 g |
| - Conservateurs | 0,2 g |
| - Eau | qsp 100 g |

Dans cet exemple, le 2,5-dihydroxy 4-méthyl phényl acétate de méthyle peut être avantageusement remplacé par 0,8 g de l'acide 2,5-dihydroxy 4-méthyl phényl hexanoïque.

EXEMPLE 2 : Emulsion eau-dans-huile

| | |
|---|---|
| - Propylène glycol | 11 g |
| - 2,5-dihydroxy 4-méthyl phényl acétate de méthyle | 0,5 g |
| - Huile minérale (vaseline) | 20,5 g |
| - Sorbitan Isostéarate (esters d'acides gras et de sorbitol) | 5 g |
| - Miglyol Gel B (hectorite modifiée par chlorure de stéaryl diméthylbenzylammonium dans le dicaprylate/dicaprate de glycéryle) | 5 g |
| - Coco-caprylate/caprate (Esters d'acides en $C_8$-$C_{10}$ et d'alcool gras de $C_{12}$-$C_{18}$) | 1 g |
| - Parfum | 0,1 g |
| - Conservateurs | 0,2 g |
| - Eau | qsp 100 g |

Dans cet exemple, le 2,5-dihydroxy 4-méthyl phényl acétate de méthyle peut être remplacé par 0,8 g de 2,5-dihydroxy 4-méthyl phényl acétate d'isoamyle.

EXEMPLE 3 : Emulsion huile-dans-eau

| | |
|---|---|
| - Ceteareth 20 (alcool cétylstéarylique oxyéthyléné 20 fois) | 1 g |
| - Glycol stéarate (palmitostéarate d'éthylène glycol) | 3 g |
| - Coco-caprylate/caprate (esters d'acides en $C_8$-$C_{10}$ et d'alcool gras de $C_{12}$-$C_{18}$) | 5 g |
| - Carbomer 934 (Polymère carboxyvinylique) | 0,3 g |
| - Triéthanolamine | 0,9 g |
| - Alcool éthylique à 96 % | 20 g |
| - 2,5-dihydroxy 4-méthyl phényl acétate de méthyle | 1,5 g |
| - Glycérine | 3 g |
| - Parfum | 0,1 g |
| - Conservateurs | 0,2 g |
| - Eau | qsp 100 g |

Dans cet exemple, le 2,5-dihydroxy 4-méthyl phényl acétate de méthyle peut être remplacé par 1 g de 2,5-dihydroxy 4-méthyl phényl acétate d'isopropyle.

EXEMPLE 4 : Lotion

| | |
|---|---|
| - Alcool éthylique à 96 % | 50 g |
| - PEG - (8 moles) (Polyéthylène glycol n° 8) | 30 g |
| - Ethoxydiglycol | 5 g |
| - Glycérine | 5 g |
| - 2,5-dihydroxy 4-méthyl phényl acétate de méthyle | 3,6 g |
| - Eau | qsp 100 g |

Dans cet exemple, le 2,5-dihydroxy 4-méthyl phényl acétate de méthyle peut être remplacé par 2,5 g de l'acide 6-(2,5-dihydroxy 4-méthoxy phényl) hexanoïque.

## Revendications

**1.** Utilisation de dérivés d'acides (2,5-dihydroxyphényl) carboxyliques, leurs homologues et leurs sels pour la préparation d'une composition cosmétique ou dermatologique ayant une action dépigmentante caractérisée par le fait que lesdits dérivés répondent à la formule suivante :

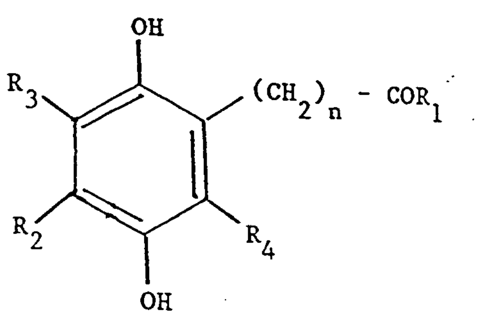

(I)

dans laquelle :
$R_1$ représente $OR_5$, OH ou

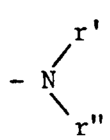

$R_5$ représente un radical alkyle en $C_1$ - $C_{20}$, linéaire ou ramifié, un radical alcényle en $C_2$-$C_{20}$ linéaire ou ramifié, un radical alkyle en $C_1$ - $C_{20}$ substitué par un ou plusieurs groupements hydroxy ou alcoxy,

r' et r'', identiques ou différents, représentent un atome d'hydrogène, un radical alkyle en $C_1$ - $C_{20}$, un radical hydroxyalkyle en $C_2$ - $C_6$ ou un radical polyhydroxyalkyle en $C_3$ - $C_6$ ou r' et r'' pris ensemble, avec l'atome d'azote, forment un hétérocycle,

le nombre d' atomes de carbone de la chaîne $-(CH_2)_n-COR_1$ étant inférieur ou égal à 21,

$R_2$ et $R_3$ identiques ou différents représentent un atome d'hydrogène, un radical alkyle en $C_1$ - $C_4$ linéaire ou ramifié ou un alcoxy en $C_1$ - $C_4$,

$R_4$ représente un atome d'hydrogène ou un radical alkyle en $C_1$ - $C_4$ linéaire ou ramifié, et

n est 0 à 20, sous réserve que lorsque $R_2$ et $R_3$ représentent un atome d'hydrogène n est supérieur ou égal à 2.

**2.** Utilisation selon la revendication 1, caractérisée par le fait que les dérivés d'acides (2,5-dihydroxyphényl)carboxyliques répondent de préférence à la formule suivante :

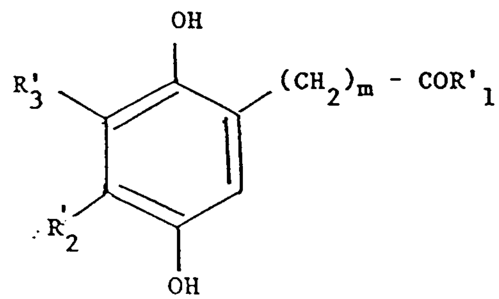

(II)

dans laquelle :

$R'_1$ a la même signification que donnée à la revendication 1 pour $R_1$,

m est 1 ou 2,

(i) lorsque m est 1, l'un au moins des radicaux $R'_2$ et $R'_3$ représente un radical alkyle en $C_1$ - $C_4$ linéaire ou ramifié, l'autre représentant éventuellement un atome d'hydrogène,

(ii) lorsque m est 2, $R'_2$ et $R'_3$, identiques ou différents, représentent un atome d'hydrogène ou un radical alkyle en $C_1$ - $C_4$ linéaire ou ramifié.

**3.** Utilisation selon la revendication 1 ou 2, caractérisée par le fait que les dérivés d'acides (2,5-dihydroxyphényl)carboxyliques, leurs homologues et leurs sels sont pris dans le groupe constitué par :

- l'acide 2,5-dihydroxyphényl propionique et ses esters éthylique et laurique,
- l'acide 2,5-dihydroxy 3,4-diméthylphényl acétique et son ester éthylique,
- le 2,5-dihydroxy 4-méthyl phényl acétate de méthyle,
- l'acide 2,5-dihydroxy 4-méthyl phényl acétique,
- l'acide 2,5-dihydroxy 4-méthyl phényl propionique et son ester éthylique,
- l'acide 2,5-dihydroxy-4-méthyl benzoïque et son ester méthylique ou éthylique,
- l'acide 2,5-dihydroxy-4-éthyl benzoïque,
- l'acide 2,5-dihydroxy-4-méthoxy benzoïque et son ester méthylique,
- l'acide 2,5-dihydroxy-4-éthoxy benzoïque,
- le 3-(2,5-dihydroxy 4'-méthyl phényl)-1-N(ω-carboxydécyl) propylamide,
- l'acide 2,5-dihydroxy 4-méthyl phényl butanoïque,
- l'acide 2,5-dihydroxy 4-méthyl phényl hexanoïque,
- l'acide 2,5-dihydroxy 4-méthoxy phényl acétique et son ester méthylique,
- le 2,5-dihydroxy 4-méthoxy benzylamide,
- l'ester méthylique de l'acide 2,5-dihydroxy 3-méthoxy phényl acétique,
- l'acide 2,5-dihydroxy 3-méthoxy phényl pentadécylique, et son ester méthylique,
- l'acide 2,5-dihydroxy phényl butanoïque et son ester méthylique,
- le 2,5-dihydroxy phényl butylamide,
- l'acide 2,5-dihydroxy phényl pentanoïque,
- le 2,5-dihydroxy phényl pentylamide,
- l'acide 2,5-dihydroxy phényl hexanoïque,

- l'acide 2,5-dihydroxy phényl octanoïque,
- l'acide 2,5-dihydroxy phényl décylique et son ester éthylique,
- l'acide 2,5-dihydroxy phényl undécylique et son ester méthylique,
- l'acide 2,5-dihydroxy-3,4-diméthyl phényl butanoïque,
- l'acide 2,5-dihydroxy-3,4-diméthoxy phényl acétique,
- l'ester éthylique de l'acide 2,5-dihydroxy 4,6-diméthyl phényl acétique,
- le 2-(2,5-dihydroxy 4-méthyl phényl)-N-octyl acétamide, et
- l'acide 6-(2,5-dihydroxy 4-méthoxy phényl) hexanoïque.

**4.** Utilisation selon l'une quelconque des revendications précédentes caractérisée par le fait que la concentration en dérivés d'acides (2,5-dihydroxyphényl)carboxyliques dans la composition est comprise entre 0,01 et 10 % en poids.

**5.** Utilisation selon l'une quelconque des revendications précédentes, caractérisée par le fait que la concentration en dérivés d'acides (2,5-dihydroxyphényl)carboxyliques dans la composition est comprise entre 0,5 et 5 % en poids.

**6.** Utilisation selon l'une quelconque des revendications précédentes caractérisée par le fait que la composition cosmétique ou dermatologique se présente sous forme d'une lotion, d'une crème, d'un lait, d'un gel, d'un masque, de microsphères ou nanosphères ou de dispersions vésiculaires.

**7.** Utilisation selon l'une quelconque des revendications précédentes, caractérisées par le fait que la composition cosmétique ou dermatologique contient en outre un humectant, un agent de surface, un kératolytique, un agent anti-inflammatoire, un agent complexant, un anti-oxydant, un conservateur, un parfum ou un filtre solaire.

**8.** Composés dérivés d'acides (2,5-dihydroxyphényl)carboxyliques caractérisés par le fait qu'ils répondent à la formule suivante :

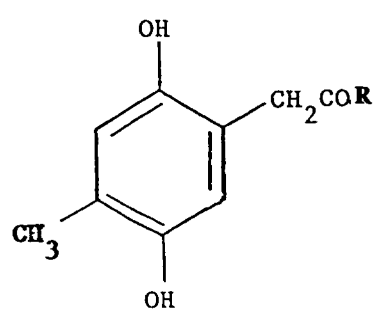

(III)

dans laquelle :
R représente le radical OR' ou NHR", R' représentant un radical alkyle inférieur en $C_1$-$C_6$, linéaire ou ramifié et R" représente un radical alkyle en $C_4$-$C_{12}$.

**9.** Composés selon la revendications 8, caractérisés par le fait qu'ils sont choisis parmi :
- le 2,5-dihydroxy 4-méthyl phényl acétate de méthyle,
- le 2,5-dihydroxy 4-méthyl phényl acétate d'éthyle,
- le 2,5-dihydroxy 4-méthyl phényl acétate de propyle,
- le 2,5-dihydroxy 4-méthyl phényl acétate d'isopropyle,
- le 2,5-dihydroxy 4-méthyl phényl acétate de butyle,
- le 2,5-dihydroxy 4-méthyl phényl acétate d'isoamyle, et
- le 2-(2,5-dihydroxy 4-méthyl phényl)-N-octyl acétamide.

## Claims

**1.** Use of derivatives of (2,5-dihydroxyphenyl)carboxylic acids, their homologues and their salts in the preparation of a cosmetic or dermatological composition having a depigmenting action, characterized in

that the said derivatives correspond to the following formula:

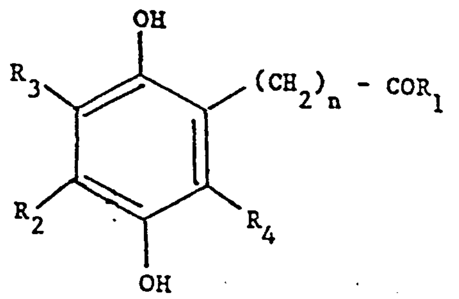

(I)

in which:

$R_1$ represents $OR_5$, OH or

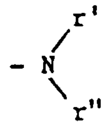

$R_5$ represents a linear or branched $C_1$-$C_{20}$ alkyl radical, a linear or branched $C_2$-$C_{20}$ alkenyl radical or a $C_1$-$C_{20}$ alkyl radical substituted by one or a number of hydroxyl or alkoxy groups,

r' and r'', which are identical or different, represent a hydrogen atom, a $C_1$-$C_{20}$ alkyl radical, a $C_2$-$C_6$ hydroxyalkyl radical or a $C_3$-$C_6$ polyhydroxyalkyl radical or r' and r'', taken together, form a heterocycle with the nitrogen atom,

the number of carbon atoms of the chain $-(CH_2)_n-COR_1$ being less than or equal to 21,

$R_2$ and $R_3$, which are identical or different, represent a hydrogen atom, a linear or branched $C_1$-$C_4$ alkyl radical or a $C_1$-$C_4$ alkoxy radical,

$R_4$ represents a hydrogen atom or a linear or branched $C_1$-$C_4$ alkyl radical and

n is 0 to 20, with the proviso that when $R_2$ and $R_3$ represent a hydrogen atom, n is greater than or equal to 2.

**2.** Use according to Claim 1, characterized in that the derivatives of (2,5-dihydroxyphenyl)carboxylic acids preferably correspond to the following formula:

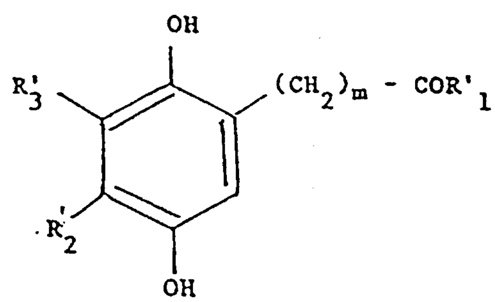

(II)

in which:

$R'_1$ has the same meaning as given in Claim 1 for $R_1$, m is 1 or 2,

(i) when m is 1, at least one of the radicals $R'_2$ and $R'_3$ represents a linear or branched $C_1$-$C_4$ alkyl radical, the other optionally representing a hydrogen atom,

(ii) when m is 2, $R'_2$ and $R'_3$, which are identical or different, represent a hydrogen atom or a linear or branched $C_1$-$C_4$ alkyl radical.

**3.** Use according to Claim 1 or 2, characterized in that the derivatives of (2,5-dihydroxyphenyl)carboxylic acids, their homologues and their salts are taken from the group consisting of:

- (2,5-dihydroxyphenyl)propionic acid and its ethyl and lauryl esters,

- (2,5-dihydroxy-3,4-dimethylphenyl)acetic acid and its ethyl ester,
- methyl (2,5-dihydroxy-4-methylphenyl)acetate,
- (2,5-dihydroxy-4-methylphenyl)acetic acid,
- (2,5-dihydroxy-4-methylphenyl)propionic acid and its ethyl ester,
- 2,5-dihydroxy-4-methylbenzoic acid and its methyl or ethyl ester,
- 2,5-dihydroxy-4-ethylbenzoic acid,
- 2,5-dihydroxy-4-methoxybenzoic acid and its methyl ester,
- 2,5-dihydroxy-4-ethoxybenzoic acid,
- 3-(2,5-dihydroxy-4-methylphenyl)-1-N-(ω-carboxydecyl)propanamide,
- (2,5-dihydroxy-4-methylphenyl)butanoic acid,
- (2,5-dihydroxy-4-methylphenyl)hexanoic acid,
- (2,5-dihydroxy-4-methoxyphenyl)acetic acid and its methyl ester,
- 2,5-dihydroxy-4-methoxybenzamide,
- methyl ester of (2,5-dihydroxy-3-methoxyphenyl)acetic acid,
- (2,5-dihydroxy-3-methoxyphenyl)pentadecanoic acid and its methyl ester,
- (2,5-dihydroxyphenyl)butanoic acid and its methyl ester,
- (2,5-dihydroxyphenyl)butanamide,
- (2,5-dihydroxyphenyl)pentanoic acid,
- (2,5-dihydroxyphenyl)pentanamide,
- (2,5-dihydroxyphenyl)hexanoic acid,
- (2,5-dihydroxyphenyl)octanoic acid,
- (2,5-dihydroxyphenyl)decanoic acid and its ethyl ester,
- (2,5-dihydroxyphenyl)undecanoic acid and its methyl ester,
- (2,5-dihydroxy-3,4-dimethylphenyl)butanoic acid,
- (2,5-dihydroxy-3,4-dimethoxyphenyl)acetic acid,
- ethyl ester of (2,5-dihydroxy-4,6-dimethylphenyl)acetic acid,
- 2-(2,5-dihydroxy-4-methylphenyl)-N-octylacetamide,
- 6-(2,5-dihydroxy-4-methoxyphenyl)hexanoic acid.

**4.** Use according to any one of the preceding claims, characterized in that the concentration of derivatives of (2,5-dihydroxyphenyl)carboxylic acids in the composition is between 0.01 and 10% by weight.

**5.** Use according to any one of the preceding claims, characterized in that the concentration of derivatives of (2,5-dihydroxyphenyl)carboxylic acids in the composition is between 0.5 and 5% by weight.

**6.** Use according to any one of the preceding claims, characterized in that the cosmetic or dermatological composition is provided in the form of a lotion, cream, milk, gel, mask, microspheres or nanospheres or vesicular dispersions.

**7.** Use according to any one of the preceding claims, characterized in that the cosmetic or dermatological composition additionally contains a humectant, a surface agent, a keratolytic agent, an antiinflammatory agent, a complexing agent, an antioxidizing agent, a preserving agent, a fragrance or a sun-screening agent.

**8.** Compounds derived from (2,5-dihydroxyphenyl)carboxylic acids, characterized in that they correspond to the following formula:

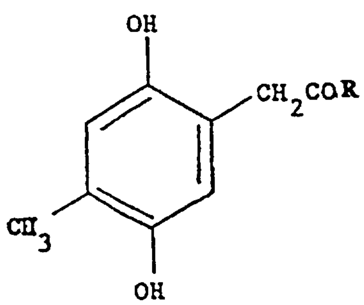

(III)

in which:
R represents the radical OR' or NHR'', R' representing a linear or branched $C_1$-$C_6$ lower alkyl radical and R'' represents a $C_4$-$C_{12}$ alkyl radical.

**9.** Compounds according to Claim 8, characterized in that they are chosen from:
- methyl (2,5-dihydroxy-4-methylphenyl)acetate,
- ethyl (2,5-dihydroxy-4-methylphenyl)acetate,
- propyl (2,5-dihydroxy-4-methylphenyl)acetate,
- isopropyl (2,5-dihydroxy-4-methylphenyl)acetate,
- butyl (2,5-dihydroxy-4-methylphenyl)acetate,
- isoamyl (2,5-dihydroxy-4-methylphenyl)acetate, and
- 2-(2,5-dihydroxy-4-methylphenyl)-N-octylacetamide.

## Patentansprüche

**1.** Verwendung von 2,5-Dihydroxyphenylcarbonsäurederivaten, deren Homologe und Salze zur Herstellung einer kosmetischen oder dermatologischen Zubereitung mit depigmentierender Wirkung, dadurch gekennzeichnet, daß die Derivate der folgenden Formel entsprechen:

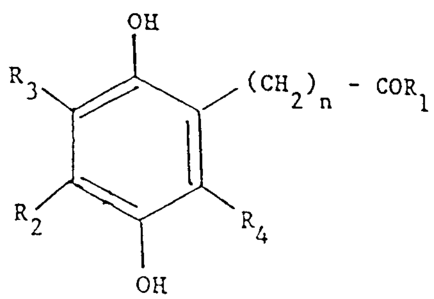

(I)

worin
$R_1$ den Rest $OR_5$, OH oder

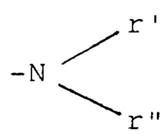

bedeutet,
$R_5$ einen gerad- oder verzweigtkettigen $C_1$-$C_{20}$-Alkylrest, einen gerad- oder verzweigtkettigen $C_2$-$C_{20}$-Alkenylrest, einen durch eine oder mehrere Hydroxy- oder Alkoxygruppen substituierten $C_1$-$C_{20}$-Alkylrest darstellt,
r' und r'', welche jeweils gleich oder verschieden sein können, ein Wasserstoffatom, einen $C_1$-$C_{20}$-Alkylrest, einen $C_2$-$C_6$-Hydroxyalkylrest oder einen $C_3$-$C_6$-Polyhydroxyalkylrest bedeuten, oder r' und r'' zusammen mit einem Stickstoffatom einen Heterocyclus bilden, wobei die Anzahl der Kohlenstoffatome in der Kette $-(CH_2)_n-COR_1$ kleiner oder gleich 21 beträgt,
$R_2$ und $R_3$, welche jeweils gleich oder verschieden sein können, ein Wasserstoffatom, ein gerad- oder verzweigtkettigen $C_1$-$C_4$-Alkylrest oder einen $C_1$-$C_4$-Alkoxyrest bedeuten,
$R_4$ ein Wasserstoffatom oder einen $C_1$-$C_4$-Alkylrest in gerad- oder verzweigtkettiger Form bedeutet, und
n = 0 bis 20 beträgt, mit der Maßgabe, daß dann, wenn $R_2$ und $R_3$ ein Wasserstoffatom darstellen, n größer oder gleich 2 ist.

**2.** Verwendung gemäß Anspruch 1, dadurch gekennzeichnet, daß die 2,5-Dihydroxyphenylcarbonsäurederivate vorzugsweise der folgenden Formel entsprechen:

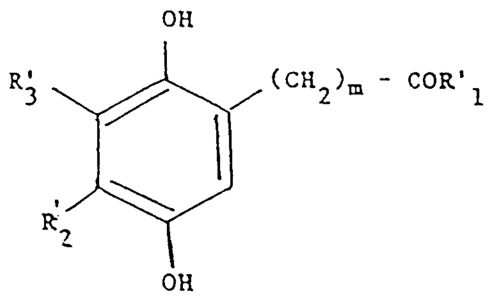

(II)

worin
R'$_1$ dieselbe Bedeutung wie in Anspruch 1 im Hinblick auf $R_1$ definiert, aufweist,
m = 1 oder 2 ist,

(i) für den Fall, daß m = 1 ist, bedeutet mindestens einer der Reste R'$_2$ und R'$_3$ einen gerade- oder verzweigtkettigen $C_1$-$C_4$-Alkylrest, wobei der andere Rest gegebenenfalls ein Wasserstoffatom darstellt,
(ii) für den Fall, daß m = 2 ist, und R'$_2$ und R'$_3$, die jeweils gleich oder verschieden sein können, ein Wasserstoffatom oder einen gerad- oder verzweigtkettigen $C_1$-$C_4$-Alkylrest bedeuten.

**3.** Verwendung gemaß Anspruch 1 oder 2, dadurch gekennzeichnet, daß die 2,5-Dihydroxyphenylcarbonsäurederivate, deren Homologe und Salze der Gruppe entnommen sind, die sich aus den folgenden Verbindungen zusammensetzt:
2,5-Dihydroxyphenylpropionsäure sowie deren Ethyl- und Laurylester,
2,5-Dihydroxy-3,4-dimethylphenylessigsäure sowie deren Ethylester,
Methyl-2,5-dihydroxy-4-methylphenylacetat,
2,5-Dihydroxy-4-methylphenylessigsäure,
2,5-Dihydroxy-4-methylphenylpropionsäure sowie deren Ethylester,
2,5-Dihydroxy-4-methylbenzoesäure sowie deren Methyl- oder Ethylester,
2,5-Dihydroxy-4-ethylbenzoesäure,
2,5-Dihydroxy-4-methoxybenzoesäure sowie deren Methylester,
2,5-Dihydroxy-4-ethoxybenzoesäure,
3-(2,5-Dihydroxy-4'-methylphenyl)-1-N-(ω-carboxydecyl)propylamid,
2,5-Dihydroxy-4-methylphenylbutansäure,
2,5-Dihydroxy-4-methylphenylhexansäure,
2,5-Dihydroxy-4-methoxyphenylessigsäure sowie deren Methylester,
2,5-Dihydroxy-4-methoxybenzylamid,
2,5-Dihydroxy-3-methoxyphenylessigsäuremethylester,
2,5-Dihydroxy-3-methoxyphenylpentadecansäure sowie deren Methylester,
2,5-Dihydroxyphenylbutansäure und deren Methylester,
2,5-Dihydroxyphenylbutylamid,
2,5-Dihydroxyphenylpentansäure,
2,5-Dihydroxyphenylpentylamid,
2,5-Dihydroxyphenylhexansäure,
2,5-Dihydroxyphenyloctansäure,
2,5-Dihydroxyphenyldecansäure sowie deren Ethylester,
2,5-Dihydroxyphenylundecansäure sowie deren Methylester,
2,5-Dihydroxy-3,4-dimethylphenylbutansäure,
2,5-Dihydroxy-3,4-dimethoxyphenylessigsäure,
2,5-Dihydroxy-4,6-dimethylphenylessigsäureethylester,
2-(2,5-Dihydroxy-4-methylphenyl)-N-octylacetamid sowie die
6-(2,5-Dihydroxy-4-methoxyphenyl)-hexansäure.

**4.** Verwendung gemaß einem der vorstehenden Ansprüche, dadurch gekennzeichnet, daß die Konzentration der Derivate von 2,5-Dihydroxyphenylcarbonsäuren in der Zubereitung zwischen 0,01 Gew.-% und 10 Gew.-% beträgt.

**5.** Verwendung gemaß einem der vorstehenden Ansprüche, dadurch gekennzeichnet, daß die Konzentration der Derivate von 2,5-Dihydroxyphenylcarbonsäuren in der Zubereitung zwischen 0,5 Gew.-% und 5 Gew.-% beträgt.

**6.** Verwendung gemäß einem der vorstehenden Ansprüche, dadurch gekennzeichnet, daß die kosmetische oder dermatologische Zubereitung in Form einer Lotion, Creme, Milch, eines Gels, einer Gesichspackung, in Form von Mikro- oder Nanokügelchen bzw. vesikulären Dispersionen vorliegt.

**7.** Verwendung gemäß einem der vorstehenden Ansprüche, dadurch gekennzeichnet, daß die kosmetische oder dermatologische Zubereitung zusätzlich noch ein Feuchthaltemittel, ein Tensid, ein Keratolytikum, ein entzündungshemmendes Mittel, ein Komplexbildner, ein Antioxidans, ein Konservierungsmittel, einen Duftstoff oder ein Sonnenschutzmittel enthält.

**8.** Von 2,5-Dihydroxyphenylcarbonsäuren abgeleitete Verbindungen, dadurch gekennzeichnet, daß sie der folgenden Formel entsprechen:

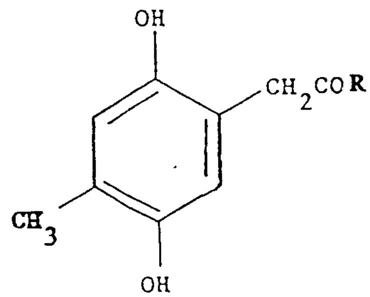

(III)

worin
R den Rest OR' oder NHR'' bedeutet, wobei R' einen niedrigen $C_1$-$C_6$-Alkylrest, der gerad- oder verzweigtkettig sein kann, bedeutet, und R'' einen $C_4$-$C_{12}$-Alkylrest darstellt.

**9.** Verbindungen gemäß Anspruch 8, dadurch gekennzeichnet, daß sie aus der folgenden Gruppe ausgewählt sind:
Methyl-2,5-dihydroxy-4-methylphenylacetat,
Ethyl-2,5-dihydroxy-4-methylphenylacetat,
Propyl-2,5-dihydroxy-4-methylphenylacetat,
Isopropyl-2,5-dihydroxy-4-methylphenylacetat,
Butyl-2,5-dihydroxy-4-methylphenylacetat,
Isoamyl-2,5-dihydroxy-4-methylphenylacetat, sowie
2-(2,5-Dihydroxy-4-methylphenyl)-N-octylacetamid.